Europäisches Patentamt

European Patent Office

Office européen des brevets

(11) Publication number: **0 218 891**
A2

# (12) EUROPEAN PATENT APPLICATION

(21) Application number: 86112247.1

(22) Date of filing: 04.09.86

(51) Int. Cl.⁴: **A61L 9/12** , A61L 9/04

(30) Priority: 15.10.85 US 787068

(43) Date of publication of application:
22.04.87 Bulletin 87/17

(84) Designated Contracting States:
CH DE FR GB IT LI NL

(71) Applicant: **Union Camp Corporation**
**1600 Valley Road**
**Wayne New Jersey 07470(US)**

(72) Inventor: **Locko, George A.**
**41 Wolfpack Road**
**Trenton New Jersey 08619(US)**

(74) Representative: **Finck, Dieter et al**
**Patentanwälte v. Füner, Ebbinghaus, Finck**
**Mariahilfplatz 2 & 3**
**D-8000 München 90(DE)**

(54) **Device for dispensing volatile substances.**

(57) A device for dispensing volatile substances, such as fragrances, insect repellents, and the like, comprised of a silicone rubber hollow body (12), and the volatile material (22) contained within the hollow - (20) is described. The volatile material (22) diffuses through the silicone rubber mass to the surrounding environment

FIG.2

EP 0 218 891 A2

# DEVICE FOR DISPENSING VOLATILE SUBSTANCES

The invention relates to a device for dispensing volatile substances.

The device is used for the slow release of a volatile substance such as a fragrance, insect repellant, deodorant, medicament and the like.

Articles of many types for the controlled release of volatile substances to the environment are well known in the art. However, all of the articles which have heretofore been disclosed in the art suffer from various disadvantages, especially with respect to performance. Ideally, an article for dispensing a fragrance or other volatile substance should dispense the volatile substance at an essentially constant rate over an extended period of time to provide an effective level of the volatile substance in the environment, up to the point at which the fragrance contained within the article is depleted.

Gel-type deodorizer dispensers in which a fragrance is dispersed in a water-based gel are generally useful in that they provide acceptable levels of fragrance to the environment. However, the gel-type systems suffer from the disadvantage that the lifetimes for fragrance release are too short, typically on the order of one or two weeks due to the rapid evaporation of water and fragrance from the gel formulations. An additional disavantage of the gel-type systems is that only certain fragrance compositions which are compatible with the water-based gels, can be used in the gel formulations.

Articles comprised of fragrances dispersed in various plastics such as polyamides, ethylene-vinyl acetate copolymer, cross-linked methacrylate derivatives, and the like have been described. Although these articles have seen use, the plastic articles suffer from the disadvantage that the levels of fragrance output are frequently too low to be effective and these articles provide non-linear release of fragrance, that is, the level of the fragrance output is typically high during the initial period of use, but then drops within a short period of time to an almost imperceptible level. An additional disadvantage of the plastic articles is that many fragrance substances are incompatible with the plastics. This frequently results in sweating of the plastic, a situation where beads of fragrance material appear on the surface of the plastic A further disadvantage of the articles in which the fragrances are dispersed in the plastics is that the liquid fragrances must either be admixed with the plastic by melt blending at high temperatures or mixed into the monomer prior to carrying out the polymerization reaction. In both instances, substantial degradation of fragrances occur. Examples of plastic articles of the aforementioned types are described in U.S.-A-4,095,031; 4,411,855; 3,926,655; 4,184,009 and CA-A-1,099,429.

JP-A-82-40,558 describes a fragrant, rubber-like molding material, formed by dispersing a fragrance in a silicone rubber and then carrying out a cross-linking reaction with an organometal salt. The articles suffer from the disadvantage that severe sweating of the silicone rubber occurs at even moderate loadings of volatile substances due to incompatibility of the silicone polymer and the volatile substances.

Wick-type deodorizers have also been described in the prior art. Although these reservoir-type systems are effective under limited conditiones, the dispensers are objectionable in appearance. The wicks are sometime prone to clogging and require special deodorants which are suitable for water-based formulations.

The U.S.-A-4,161,283 describes a fragrance-dispensing pouch which is comprised of a fragrance-containing reservoir defined by an outer wall made of a fragrance-permeable film, which is heat sealed to an impermeable inner wall by an adhesive substance. This device suffers from the disadvantage that it is not self-supporting and therefore cannot be conveniently located where desired. It is also difficult to manufacture and an article of this type cannot be made into a variety of desirable shapes.

In view of the foregoing, the object of this invention is to provide a self-supporting device for dispensing or releasing volatile substances from within the device to the surrounding environment at a relatively constant rate and at an effective level until the point in time at which the volatile substance contained within the device has been essentially completely released.

According to the invention, this object is obtained with a device for dispensing volatile substances comprising a silicone rubber, hollow body containing in the hollow thereof the liquid volatile substance which is dispensed by diffusing through the silicone rubber.

Preferably the body is reinforced with a screen reinforcement.

Advantageously the hollow body is a tubular body having a first closed end and a second open end closed by a.closure member. Conveniently the closure member is a removable plug.

The device may have an outer shape of a figurine. The volatile substance-dispensing device according to the invention can be made in a variety of shapes, can release almost all of the volatile substance, results in a cost-effective use of the volatile substance, and can be used with almost any fragrance formulation or other volatile substance, including expensive perfumes which can be used without any alteration of composition or aroma character of the volatile substance, since the volatile substance is added to the reservoir after the hollow body has been made. Further the reservoir can be refilled with either the same or another volatile substance after the original volatile substance has been depleted. This is attractive to the typical consumer due to its versatility and a perceived cost savings. It is attractive to the supplier of the volatile substance, for example, a fragrance house, because it maximizes resale of their product to the original consumer.

For a better understanding of the invention as well as other objects and further features thereof, reference is made to the following detailed description to be read in conjunction withthe accompanying drawings, wherein:

Fig. 1 is a perspective view of the silicone rubber figurine made in accordance with the invention;

Fig. 2 is an axial-sectional view along lines 2-2 of the silicone rubber figurine depicted in Fig. 1;

Fig. 3 is a cross-sectional side view of a hollow silicone rubber figurine, wherein the walls are reinforced by a wire or plastic screen.

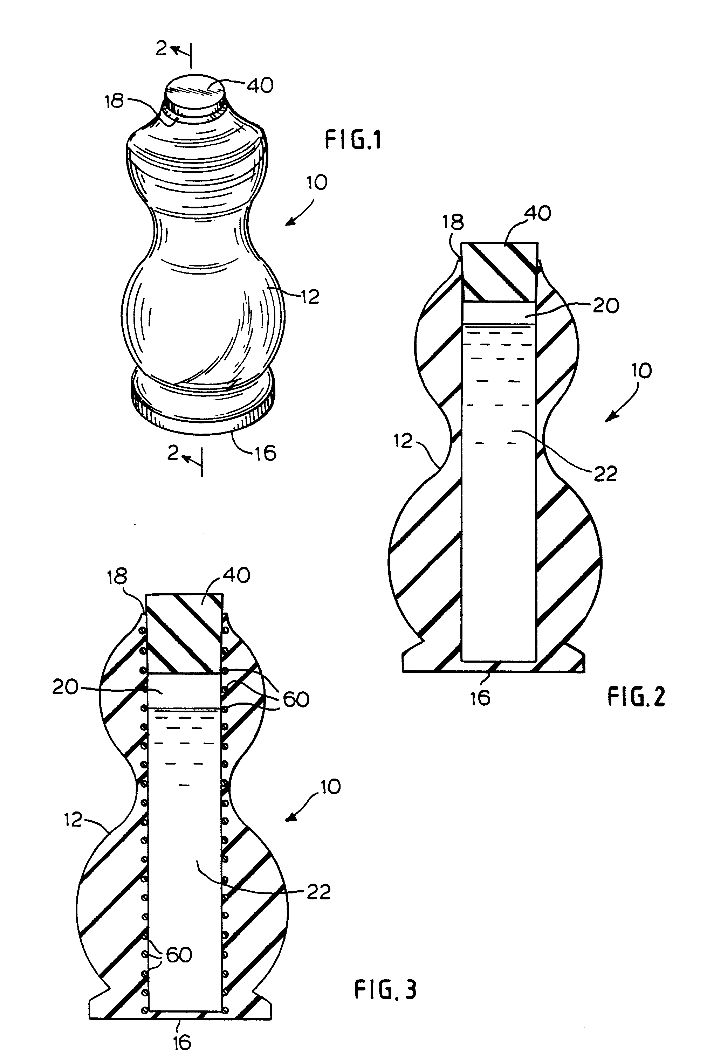

In Fig. 1 a hollow, silicone-rubber figurine 10 is shown which is representative of the articles of the invention. The figurine 10 has a generally tubular body 12 having a first, closed end 16 and a second, open end 18.

As shown in Fig. 2 the body 12 together with ends 16, 18 define a hollow or chamber 20 containing a volatile liquid substance 22. The open end 18 of the body 12 is closed by a closure member 40 thereby completely enclosing the chamber 22. The closure member 40 may be a plug made of silicone rubber or material such as polyethylene, polyvinyl chloride, or the like inserted into the open end 18 of the hollow body 12 to provide a completely closed reservoir (chamber 20). Optionally, the member 40 can be made to be removed so that the reservoir can be refilled after depletion of the volatile substance 22, as described more fully hereinafter.

The body 12 of figurine 10 is made of silicone rubber, permeable to the volatile substance 22. The use of silicone rubbers is particularly important, because the high gas permeability of silicone rubber allows for rapid release of the volatile substances 22 from within the hollow body 12. A silicone rubber is herein defined as a cross-linked silicone elastomer of the type vulcanized at room temperature (RTV) or at elevated temperatures - (HTV). Dimethyl siloxanediol with silicone resin or alkyl silicate as cross-linking agents are typically used. Typically, fillers such as silica, calcium carbonate, titanium oxide, and the like may be added to the polymer formulation, usually by the manufacturer of the silicone polymer The filler materials provide rigidity.

One example of a preferred silicone rubber used in the invention is a RTV silicone rubber. This material can be cross-linked at room temperature with a RTV catalyst. When allowed to cure in a suitable mold, this material will give a hollow body 12 which can then be loaded with a liquid volatile substance 22 and used as described herein.

Silicone rubber, devices of this invention may also be made by injection molding processes. An injection molding process is particularly advantageous because it can be automated and will allow for the cost effective rapid production of the devices described herein.

Typically, the silicone rubber devices of the invention are designed to contain between about 1 to 50 g of a liquid volatile substance, preferably between about 3 to 25 g.

Unexpectedly, it has been found that the silicone rubber wall thickness of body 12 is not a limiting variable, in that hollow bodies having very thick walls, as thick as about 5 mm to about 15 mm, will give uniform and high rates of output of volatile substances. Variable wall thickness, such as those depicted for the figurine in Fig. 1 and 2 will also give acceptable results. Hollow bodies 12 having walls as thin as about 2 to 3 mm, may be used in the articles of the invention. However, as wall thickness is reduced, additional materials, for example, wire or plastic screen, may be needed to provide the hollow body with additional rigidity.

Fig. 3 is a cross-sectional side elevation of an alternative embodiment device 10 of the invention wherein features similar to the structural features of the figurine shown in Figures 1 and 2 are assigned numerical identifications identical to those of the Fig. 1 and 2. The difference between the device of Fig. 1 and 2 and the device of Fig. 3 is found in the presence of a wire screen 60 on the inner aspect of the walls of body 12, as a means of giving additional rigidity and wall support. The wire or plastic screen or other reinforcing material 60 may be either imbedded in the silicone rubber matrix or inserted inside the hollow body 12, butted against the inner wall of the body 12 as depicted in Fig. 3. An advantage of the use of a reinforcing material 60 is that it will reduce the cost of the silicone rubber by allowing the use of lesser amounts of the silicone rubber (a thinner wall).

**Claims**

1. Device for dispensing volatile substances comprising a silicone rubber, hollow body (12) containing in the hollow (20) thereof the liquid volatile substance (22) which is dispensed by diffusing through the silicone rubber.

2. Device according to claim 1 wherein the body (12) is reinforcedwith a screen reinforcement (60).

3. Device according to claim 1 or 2 wherein the hollow body (12) is a tubular body having a first closed end (16) and a second open end (18) closed by a closure member (40).

4. Device according to claim 3 wherein the closure member (40) is a removable plug.

5. Device according to one of the claims 1 to 5 characterized by an outer figurine shape (10).

FIG.1

FIG.2

FIG. 3